(19) Europäisches Patentamt
European Patent Office
Office européen des brevets

(11) Veröffentlichungsnummer : **0 434 809 B1**

(12) **EUROPÄISCHE PATENTSCHRIFT**

(45) Veröffentlichungstag der Patentschrift :
05.10.94 Patentblatt 94/40

(51) Int. Cl.$^5$ : **G01N 33/543**, G01N 33/68,
C12N 5/18

(21) Anmeldenummer : **90910666.8**

(22) Anmeldetag : **17.07.90**

(86) Internationale Anmeldenummer :
**PCT/EP90/01170**

(87) Internationale Veröffentlichungsnummer :
**WO 91/01499 07.02.91 Gazette 91/04**

(54) VERFAHREN ZUM NACHWEIS VON LEBERZIRRHOSE.

(30) Priorität : **19.07.89 DE 3923951**

(43) Veröffentlichungstag der Anmeldung :
**03.07.91 Patentblatt 91/27**

(45) Bekanntmachung des Hinweises auf die
Patenterteilung :
**05.10.94 Patentblatt 94/40**

(84) Benannte Vertragsstaaten :
**AT BE CH DE DK ES FR GB IT LI LU NL SE**

(56) Entgegenhaltungen :
EP-A- 267 355
EP-A- 267 356
US-A- 4 727 021
US-A- 4 775 620
DIALOG INFORMATIONAL SERVICE, file 154
medline; M.B. RAY, acc. no. 06161628; and P.
VAN EYKEN et al., acc. no. 06870849
LABORATORY INVESTIGATION, vol. 55, no. 4,
1986; M. OSBORN et al., pp. 497-498
VIRCHOW's ARCHIV A (Pathol. Anat.), vol. 413,
1988; D. BROEKAERT et al., pp. 39-51
EMBO JOURNAL, vol. 1, no. 11, 1982; O. GIGI
et al., pp. 1429-1437

(56) Entgegenhaltungen :
BREAST CANCER RESEARCH & TREATMENT,
vol. 14, 1989; M. FERRERO et al., p. 166
CELL TISSUE RES., vol. 254, 1988; W. SAVINO
et al., pp. 225-231
JOURNAL INVEST. DERMATOL., vol. 91, no. 6,
December 1988; I. KUROKAWA et al., pp.
566-571

(73) Patentinhaber : **BOEHRINGER MANNHEIM
GMBH**
**D-68298 Mannheim (DE)**

(72) Erfinder : **BODENMÜLLER, Heinz, Dr.**
**Zugspitzstrasse 1**
**D-8132 Tutzing (DE)**
Erfinder : **DESSAUER, Andreas, Dr.**
**Herre-Strasse 1**
**D-8132 Tutzing (DE)**

(74) Vertreter : Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B. Böhm
Postfach 86 08
20
D-81635 München (DE)

**Beschreibung**

Die Erfindung betrifft ein Verfahren und ein Reagenz zum Nachweis von Leberzirrhose.

Die Diagnose von Lebererkrankungen ist schwierig, da die meisten Lebererkrankungen nur sehr unspezifische Symptome haben. Insbesondere bereitet die Abgrenzung der einzelnen Lebererkrankungen wie Hepatitis, Leberzirrhose und Lebercarzinom Schwierigkeiten. Verläßliche Befunde können häufig nur durch invasive Methoden erhalten werden, was für den Patienten belastend ist. Die Bestimmung von in der Leber gebildeten Enzymen wie Transaminasen, Cholinesterase sowie von Bilirubin gibt nur allgemeine Hinweise und ist für eine differenzierte Diagnose nicht brauchbar.

EP-A-0 267 355 beschreibt ein Verfahren zum Identifizieren gewebstypischer, unlöslicher Cytoskelettproteine, bei dem ein erster monoklonaler "Fänger"-Antikörper, der gegen ein erstes Epitop aller Cytokeratinfragmente gerichtet ist, und ein zweiter monoklonaler "Detektor"-Antikörper, der gegen ein zweites Epitop gerichtet ist, das spezifisch für das jeweilige Cytokeratin ist, verwendet wird. Die Verwendung von für einzelne Cytokeratine spezifischen Antikörpern zum Nachweis der Leberzirrhose wird nicht offenbart.

P. van Eyken (1988) Histopathology 13, 605-617 offenbart, daß bei Alkohol-Leber-Erkrankungen Mallory-Körperchen (Cytokeratin-Aggregate) unterschiedlicher Zusammensetzung auftreten. In dieser Arbeit wird jedoch nicht offenbart, daß das Auftreten der Mallory-Körperchen mit dem Auftreten von Leber-Zirrhose korreliert sein könnte.

Es besteht daher ein Bedarf nach einem Nachweisverfahren, mit dem eine Leberzirrhose eindeutig und in einfacher Weise nachgewiesen werden kann, wobei andere Lebererkrankungen, wie insbesondere Hepatitis und Lebercarzinom, nicht erfaßt werden.

Es war daher Aufgabe der vorliegenden Erfindung, ein Verfahren zum spezifischen Nachweis von Leberzirrhose zur Verfügung zu stellen, das ohne den Patienten belastende Eingriffe einfach und schnell im Labor durchgeführt werden kann.

Diese Aufgabe wird gelöst durch ein Verfahren zum Nachweis von Leberzirrhose, das dadurch gekennzeichnet ist, daß man die Probe einer Körperflüssigkeit mit mindestens zwei Rezeptoren $R_1$ und $R_2$ inkubiert, von denen $R_1$ die Bindung an eine feste Phase vermittelt und $R_2$ markiert ist, wobei entweder $R_1$ oder $R_2$ einen monoklonalen Antikörper spezifisch für Cytokeratin 5 bzw. 7 und der andere der beiden Rezeptoren $R_2$ oder $R_1$ einen monoklonalen Antikörper spezifisch für CK 19 bzw. 18 enthält, oder beide Rezeptoren einen monoklonalen Antikörper spezifisch für CK 8 enthalten, die feste von der flüssigen Phase trennt und die Markierung in einer der beiden Phasen mißt.

Cytokeratine (CK) sind als Intermediärfilament-Proteine Bestandteile des Cytoskelettes von Epithelzellen. Es sind 19 Cytokeratine bekannt, von denen die Cytokeratine 1 bis 8 als basische und die Cytokeratine 9 bis 19 als saure Cytokeratine bezeichnet werden. Die Cytokeratine können sich in der Zelle zu Tetrameren zusammenlagern. Ein Tetramer besteht dabei aus jeweils zwei basischen und zwei sauren Cytokeratinmolekülen. Durch lineare Aggregation von Tetrameren entstehen Filamente. Intakte Cytokeratinmoleküle sind als integrale Bestandteile der Intermediärfilamente epithelialer Zellen wasserunlöslich. Die Komplexität und Zusammensetzung der Cytokeratine ist unterschiedlich in den verschiedenen epithelialen Geweben, d.h. Epithelzellen unterschiedlicher Gewebeherkunft haben eine unterschiedliche Cytokeratinzusammensetzung. Überraschenderweise wurde nun festgestellt, daß ein erhöhter Gehalt an löslichen Formen von Aggregaten der Cytokeratine CK 5 bzw. 7 in Verbindung mit CK 19 bzw. 18 oder von CK 8 in einer Körperflüssigkeit mit dem Auftreten von Leberzirrhose korreliert.

Erfindungsgemäß wird das Vorliegen einer Leberzirrhose nachgewiesen, indem man in einer Körperflüssigkeit bestimmt, ob Bindungsstellen sowohl für Antikörper gegen Cytokeratin 5 bzw. 7 als auch für Antikörper gegen Cytokeratin 19 bzw. 18 oder mehrere Bindungsstellen für CK 8 vorhanden sind, bzw. ob die Anzahl der Bindungsstellen gegenüber der Körperflüssigkeit von Gesunden erhöht ist. Bevorzugt für das erfindungsgemäße Verfahren ist es, daß entweder ein monoklonaler Antikörper gegen Cytokeratin 5 in Verbindung mit einem monoklonalen Antikörper gegen Cytokeratin 19, ein monoklonaler Antikörper gegen Cytokeratin 7 in Verbindung mit einem monoklonalen Antikörper gegen Cytokeratin 18 oder zwei monoklonale Antikörper gegen Cytokeratin 8 verwendet werden. Dazu inkubiert man die Probelösung, die eine Körperflüssigkeit wie Serum, Urin oder Lymphe sein kann und bevorzugt Serum ist, mit mindestens zwei Rezeptoren $R_1$ und $R_2$, von denen $R_1$ die Bindung an eine feste Phase vermittelt und $R_2$ markiert ist. Dabei enthält einer der beiden Rezeptoren $R_1$ oder $R_2$ einen monoklonalen Antikörper gegen Cytokeratin 5, 7 oder 8. Unter monoklonalem Antikörper werden dabei sowohl vollständige Antikörper als auch Antikörperfragmente, die die Antigen-Bindungsstellen für die jeweiligen Cytokeratine aufweisen, verstanden.

Rezeptor $R_1$ vermittelt die Bindung an die feste Phase. Dazu kann Rezeptor $R_1$ entweder direkt an eine feste Phase gebunden sein oder aber immobilisierbar sein. In einer bevorzugten Ausführungsform ist Rezeptor $R_1$ ein Konjugat aus einem monoklonalen Antikörper und einer spezifisch bindefähigen Substanz. Der mit der spezifisch bindefähigen Substanz bindefähige Partner ist an eine Festphase gebunden. Als spezifisch bindefähige Paare können beispielsweise Antigen-Antikörper; Hapten-Antikörper; Biotin-Antibiotin-Antikörper; Biotin-Avidin; Biotin-Streptavidin; Protein A-Immun-$\gamma$-Globulin genannt werden. Besonders bevorzugt wird in dieser Ausführungsform als $R_1$ ein Konjugat des monoklonalen Antikörpers mit Biotin und als Festphase eine Matrix verwendet, die an ihrer Oberfläche Streptavidin trägt. Durch Bindung des Biotins mit dem Streptavidin erfolgt dann die Immobilisierung des monoklonalen Antikörpers. Bevorzugt ist auch eine Ausführungsform, bei der an der Oberfläche der festen Phase Antikörper gegen den Fc-Teil der verwendeten monoklonalen Antikörper oder Protein-A-Moleküle gebunden werden, wobei dann durch Bindung der Fc-Teile der monoklonalen Antikörper die Immobilisierung erfolgt.

In einer weiteren bevorzugten Ausführungsform sind an eine Matrix Biotinmoleküle gebunden und als Re-zeptor $R_1$ wird ein Konjugat aus Biotin und dem monoklonalen Antikörper verwendet. Die Immobilisierung kann dann nach Durchführung der immunologischen Reaktion durch Zugabe von Streptavidin erfolgen.

Als feste Phase sind die in immunologischen Verfahren üblicherweise verwendeten Materialien geeignet. Beispielsweise können Polymermaterialien oder auch cellulosehaltige Materialien sowie Glas verwendet werden. Als besonders geeignet haben sich Polystyrol, Polymethacrylat, Teflon, Polyamid, Copolymere aus Styrol und Acrylnitril, Glas- und Celluloseprodukte erwiesen. Die Matrix kann in beliebiger Form vorliegen, z.B. als Röhrchen, Mikrotiterplatte, Kugel, Film, Pulver, Körnchen oder Faservlies. Geeignet sind beispielsweise nach einem in der DE-A 36 40 412 beschriebenen Verfahren erhaltene Festphasen.

Der Rezeptor $R_2$ enthält jeweils den anderen monoklonalen Antikörper, der erfindungsgemäß notwendig ist, d.h. wenn für den Rezeptor $R_1$ Anti-CK 5-Antikörper verwendet wird, enthält Rezeptor $R_2$ einen monoklo-nalen Antikörper gegen CK 18 oder 19, vorzugsweise gegen CK 19 und umgekehrt. Dasselbe gilt für das Paar Cytokeratin 7/Cytokeratine 18 und 19. Hier ist die Kombination CK 7 mit CK 18 bevorzugt. Ebenso ist die Kombination CK 8 mit CK 8 bevorzugt. Die Markierung des Rezeptors $R_2$ erfolgt nach bekannten Methoden. Als Markierung sind radioaktive Substanzen, Enzyme, fluoreszierende und chemilumineszierende Substanzen geeignet. Der Nachweis der Markierung erfolgt dabei nach bekannten Methoden. Bevorzugt wird als Markierung ein Enzym eingesetzt. Als Enzyme geeignet sind insbesondere Peroxidase, alkalische Phosphatase und $\beta$-Galactosidase. Der Nachweis des Enzyms erfolgt durch Zugabe eines Substrats und Messung der gebildeten Farbe.

Für die Durchführung des erfindungsgemäß vorgesehenen immunologischen Bestimmungsverfahrens sind sehr viele Varianten bekannt, die alle hier geeignet sind. So können zwei oder drei oder auch mehr Rezeptoren verwendet werden und die Inkubation mit den einzelnen Rezeptoren kann in verschiedener Reihenfolge in homogener oder heterogener Phase erfolgen. Diese Verfahrensvarianten sind dem Fachmann bekannt und bedürfen hier keiner näheren Erläuterung. Bevorzugt erfolgt die erfindungsgemäße Bestimmung als Sandwich-Immunoassay. Dazu wird Rezeptor $R_1$ immobilisiert oder immobilisierbar gemacht und mit der Probelösung umgesetzt. Anschließend wird Rezeptor $R_2$ zugegeben. Es bilden sich Komplexe aus dem immobi-lisierten Rezeptor $R_1$, der nachzuweisenden Substanz sowie Rezeptor $R_2$. Nur Komplexe, die an die Festphase gebunden sind und eine Markierung tragen, gehen in die Auswertung ein.

Mit dem erfindungsgemäßen Verfahren kann das Vorhandensein von Bindungsstellen für Antikörper gegen CK 5 und CK 18/CK 19, Antikörper gegen CK 7 und CK 18/CK 19 oder Antikörper gegen CK 8 und CK 8 schnell, einfach und reproduzierbar nachgewiesen werden. Eine erhöhte Konzentration dieser Cytokeratine bzw. von Substanzen, die antigene Determinanten aufweisen, die zu einer Bindung mit den entsprechenden monoklonalen Antikörpern führen, ist im wesentlichen nur nachweisbar bei Vorliegen einer Leberzirrhose. Es konnte gezeigt werden, daß bei anderen Lebererkrankungen wie Hepatitis oder Fettleber oder anderen Erkrankungen, die mit Lebererkrankungen in Verbindung stehen wie Pankreatitis oder primäre biliäre Zirrhose eine derartige Erhöhung in der Regel nicht auftritt, so daß mit dem erfindungsgemäßen Verfahren ein spezifischer Nachweis möglich ist.

Ein weiterer Gegenstand der Erfindung ist ein Reagenz zum Nachweis von Leberzirrhose, das dadurch gekennzeichnet ist, daß es mindestens zwei Rezeptoren $R_1$ und $R_2$ enthält, von denen $R_1$ die Bindung an eine feste Phase vermittelt und $R_2$ markiert ist, wobei entweder $R_1$ oder $R_2$ einen monoklonalen Antikörper spezi-fisch für CK 5 bzw. CK 7 und der andere der beiden Rezeptoren $R_2$ oder $R_1$ einen monoklonalen Antikörper spezifisch für CK 19 bzw. CK 18 enthält, oder beide Rezeptoren einen monoklonalen Antikörper spezifisch für CK 8 enthalten.

Bevorzugt enthält das erfindungsgemäße Reagenz einen an eine Festphase gebundenen oder immobilisierbaren monoklonalen Antikörper gegen Cytokeratin 5 und einen markierten monoklonalen Antikörper gegen Cytokeratin 19.

Ebenso bevorzugt sind die Kombinationen CK 7 mit CK 18 und CK 8 mit CK 8.

Gegenstand der Erfindung ist auch die Verwendung der bei der European Collection of Animal Cell Cultures, Porton Down (GB), hinterlegten Zellkulturen ECACC 89030306 und ECACC 89030305 (CK 5), ECACC 89030301 (CK 7), ECACC 89030303 (CK 18), ECACC 89030304 (CK 19) und ECACC 89030302 (CK 8), die Antikörper gegen die entsprechenden Cytokeratine produzieren, in einem erfindungsgemäßen Verfahren zur Bestimmung von Leberzirrhose.

Die Erfindung wird durch die folgenden Beispiele erläutert.

**Beispiel 1**

Gewinnung von monoklonalen Antikörpern gegen Cytokeratine

Balb/c-Mäuse werden mit gereinigtem Cytokeratin oder Cytokeratinfragmenten in komplettem Freundschen Adjuvans erstimmunisiert. Nach weiteren Immunisierungen im inkompletten Freundschen Adjuvans werden nach 3 bis 4 Monaten die B-Lymphozyten der immunisierten Tiere mit einer geeigneten Myelomzellinie fusioniert. Die hierbei gewonnenen Hybridomzellinien werden mit den dazu üblichen Hilfsmitteln kloniert (z.B. fluorescence activated cell sorter) und mit Hilfe geeigneter Methoden selektioniert (z.B. ELISA). Auf diese Weise können monoklonale Antikörper gewonnen werden, die spezifisch gegen bestimmte Cytokeratine gerichtet sind.

Es werden 5 monoklonale Antikörper mit verschiedenen Spezifitäten beschrieben (siehe Tabelle 1). An einem repräsentativen Beispiel werden die Immunisierung, die Klonierung und die Spezifitätsbestimmung detailliert beschrieben. Die anderen vier monoklonalen Antikörper werden analog hergestellt.

**Beispiel 2**

Monoklonaler Antikörper gegen CK 5

Isolierung des Antigens CK 5

Das mehrfach geschichtete, nicht verhornende Plattenepithel der menschlichen Gaumenmandel wird durch einen Hitzeschritt (5 min/60°C) von der bindegewebigen Unterlage (Basalmembran) abgetrennt und nach mehreren Waschungen homogenisiert.

Der 1 % Triton K-100/Kochsalz-resistente Rückstand aus dem homogenisierten Plattenepithel wird in 8 mol/l Harnstoff/20 mmol/l Tris HCl, pH 7,5/30 mmol/l KCl/0,2 % (v/v) Mercaptoethanol lysiert und der Überstand auf eine Chromatographie-Säule (DEAE-Sepharose® fastflow) gegeben. Die Säule (15x16cm) wird bei Raumtemperatur und einer Flußrate von 18 ml/Stunde mit einem von 30 bis 170 mmol/l ansteigenden KCl-Gradienten entwickelt über 14 Stunden. Der Proteingehalt jeder Fraktion wird durch Elektröphorese geprüft. Die CK 5 enthaltende Fraktion wird über das Molekulargewicht (ca. 58000) bzw. den isoelektrischen Punkt (ca. pH 7,5) identifiziert. Die lyophilisierte CK 5 haltige Fraktion wird in 9,5 mol/l Harnstoff/0,2 % (v/v) Mercaptoethanol/20 mmol/l Tris HCl/1 mmol/l KCl aufgenommen und in einer Chromatographiesäule (DEAE-Sepharose® fastflow; 15,5x1,6cm) durch Anwendung eines 1 bis 180 mmol/l linear ansteigenden KCl-Gradienten weitergereinigt. Die Identifikation von CK 5 erfolgt über das Molekulargewicht bzw. den isoelektrischen Punkt.

Immunisierung

Balb/c-Mäuse, 8 bis 12 Wochen alt, werden mit 100 µg CK 5-Antigen in komplettem Freundschen Adjuvans intraperitoneal immunisiert. In vierwöchigem Rhythmus werden zwei weitere Immunisierungen mit jeweils 50 µg Antigen in inkomplettem Freundschen Adjuvans durchgeführt.

Fusion und Klonierung

Milzzellen einer immunisierten Maus werden mit Ag8.653 Myelomzellen (ATCC-CRL 8375) im Verhältnis 1:1 nach dem Standardverfahren gemäß J. of Immunol. Meth., Vol. 39, 285-308 fusioniert. Die Fusionsprodukte werden auf 24er Kulturschalen gemeinsam mit $5 \times 10^4$ Peritoneal-Exudat-Zellen als Futterzellen angesät. Positive Primärkulturen werden 2 bis 3 Wochen nach Fusion mit Hilfe eines Fluorescence activated cell sorters kloniert. Die Zellen werden einzeln in 96-well-Zellkulturplatten abgelegt und mit HECS gefüttert. Als Kulturmedium wird handelsübliches RPMI 1640 Medium mit 10 % fötalem Kälberserum verwendet (nach J.A.M.A. 199, 519 (1957).

Induktion von Ascites

2 bis 5 x 10⁶ Hybridzellen werden intraperitoneal in mit Pristan vorbehandelte Mäuse gespritzt. Nach 10 bis 15 Tagen kann Ascites mit einer Antikörperkonzentration von 5 bis 10 mg/ml gewonnen werden.

Bestimmung der Spezifität

1. ELISA: Um die Anwesenheit und Spezifität von Antikörpern zu erkennen, wird ein Enzyme-linked immuno-sorbent assay (ELISA) angewandt. Dazu werden auf ELISA-Platten pro well 80 µl Cytokeratinantigen in Carbonatpuffer vorgelegt. Anschließend wird mit 80 µl Hybridomakulturüberstand inkubiert. Danach wird eine Stunde mit gegen Mausimmunglobulin gerichteten Antikörpern, die aus Schaf stammen und mit POD (Peroxidase) markiert sind, inkubiert. Zum Nachweis von gebundenem Enzym wird ABTS (2,2'-Azino-di-[3-ethyl-benzthiazolin-sulfonsäure(6)]-di-ammoniumsalz) als Substrat zugegeben. Nach 20 Minuten werden die Extinktionen in einem Photometer bei 405 nm bestimmt.

2. Immunoblot ("Western-Blot"): Aus humanem nicht verhornendem Plattenepithel (enthält die CK 4, 5, 6, 13, 14) sowie aus humaner Epidermis (enthält die CK 1, 5, 10, 14) werden Cytoskelettpräparationen hergestellt und auf SDS-Polyacrylamid-Gelelektrophorese aufgetrennt. Nach dem Transfer der Proteine auf Nitrocellulose werden durch Inkubation mit den einzelnen Antikörpern die Reaktivitäten derselben untersucht.

3. Immunhistochemie/Immuncytochemie: Gefrierschnitte der Gewebe Leber (CK 8, 18), Darm (CK 8, 18, 19) und Endometrium (CK 7, 8, 18, 19) sowie methanolfixierte Zellen der Linien MCF-7 (ATCC HTB22) (CK 8, 18, 19), Hela (ATCC CCL2) (CK 7, 8, 18, 19), A-431 (ATCC CRL1555) (CK 5,-8, 13, 15, 17, 18) und RT-112 (CK 7, 8, 13, 17, 18, 19) werden mit Hybridomakulturüberständen inkubiert und die gebundenen Antikörper mit Schafantikörpern gegen Maus-Immunglobulin, die mit FITC (Fluoresceinisothiocyanat) markiert sind, sichtbar gemacht.

## T a b e l l e   1

| MAK | Immunogen | Spezifität | Subklasse | Referenz |
|-----|-----------|-----------|-----------|----------|
| ECACC 89030301 | Cytoskelett von RT 112-Zellen | CK 7 | IgG 1 | 1), 3) |
| ECACC 89030303 | CK 18 (45 kD) aus MCF-7-Zellen | CK 18 | IgG 1 | 2), 3), 4), 5) |
| ECACC 89030306 89030305 | CK 5 aus hu Mundschleim-haut | CK 5 | IgG 1 | 1), 3), 6), 7) |
| ECACC 89030304 | Cytoskelett von MCF-7-Zellen | CK 19 | IgG 1k | 2), 3) |
| ECACC 89030302 | Cytoskelett von MCF-7 Zellen | CK 8 | IgG 1 | 2), 3) |

1) Eur. J. Cell Biol. 38 (1985) 234-244
2) Lab. Invest. 55 (1986) 497-504
3) Dissertation T. Achtstätter, Universität Heidelberg 1988
4) Am. J. Pathol. 114 (1981) 121
5) EMBO J. 1 (1982) 1641
6) Intermediate Filaments, Vol. 455, E. Wang, D. Fischman, R.K.H. Liem, T.-T. Sun (eds.) The New York Academy of Science (1985) S. 282-306
7) TIG, Vol. 4 (1988) 277-281

**Beispiel 3**

Es wurden Seren von Patienten mit unterschiedlichen, sowohl malignen als auch benignen epithelialen Krankheiten untersucht. Tabelle 2 stellt die Ergebnisse dar.

Tabelle 2

| | positiv/total | | | | | |
|---|---|---|---|---|---|---|
| | Nachweis von CK 5/19 | | Nachweis von CK 7/18 | | Nachweis von CK 8/8 | |
| | CK 5/19 positive Seren | unter- suchte Seren | CK 7/18 positive Seren | unter- suchte Seren | CK 8/8 positive Seren | unter- suchte Seren |
| <u>Gesunde</u> | 0 | 6 | 0 | 6 | 0 | 6 |
| <u>Benigne Krankheiten</u> | | | | | | |
| Leberzirrhose | 5 | 5 | 4 | 5 | 4 | 6 |
| Hepatitis | 0 | 5 | 0 | 5 | 0 | 3 |
| Fettleber | 0 | 9 | 0 | 9 | – | – |
| prim. bil. Zirrhose | 0 | 3 | 0 | 3 | 0 | 3 |
| Niereninsuffizienz | 1 | 3 | 1 | 3 | 1 | 3 |
| Pankreatitis | 0 | 2 | 0 | 2 | 1 | 2 |
| <u>Karzinome</u> | | | | | | |
| Leber | 1 | 5 | 0 | 5 | 1 | 3 |
| Bronchial | 0 | 6 | 0 | 6 | 0 | 3 |
| Mamma | 0 | 3 | 0 | 3 | 0 | 3 |
| Ovarial | 0 | 3 | 1 | 3 | 0 | 3 |
| Colon | 0 | 3 | 0 | 3 | 1 | 3 |
| Pankreas | 0 | 3 | 0 | 3 | 0 | 3 |
| Magen | 0 | 3 | 0 | 3 | 0 | 3 |
| Collum | 0 | 2 | 0 | 2 | 0 | 3 |

Wie die Tabelle zeigt, gelingt der Nachweis der Kombination Cytokeratin 5/19, 7/18 oder 8/8 nur bei Patienten, bei denen eine Leberzirrhose vorliegt. Die positive Reaktion des Patienten mit Niereninsuffizienz ist darauf zurückzuführen, daß der Patient zusätzlich eine beginnende oder ausgebildete Leberzirrhose hatte. Auch bei dem Patienten mit Leberkarzinom, bei dem ein positiver Befund festgestellt wurde, ist dies darauf zurückzuführen, daß die Leber zirrhoseartig verändert war.

Die hinterlegten Zellinien ECACC 89030301, 89030302, 89030303, 89030304 und 89030306 wurden alle am 3. März 1989 bei PHLS Centre for Applied Microbiology & Research, Porton Down, Salisbury, Wilts. SP4 OJG, U.K., unter der jeweiligen oben angegebenen Nummer hinterlegt. Die Zellinie ATCC CCL 77 wurde am 12. Oktober 1983 bei der American Type Culture Collection, 12301 Parklawn Drive, Rockville, Maryland 20852, USA, hinterlegt.

**Patentansprüche**

1. Verfahren zum Nachweis von Leberzirrhose,
   **dadurch gekennzeichnet,**
   daß man die Probe einer Körperflüssigkeit mit mindestens zwei Rezeptoren $R_1$ und $R_2$ inkubiert, von denen $R_1$ die Bindung an eine feste Phase vermittelt und $R_2$ markiert ist, wobei entweder $R_1$ oder $R_2$ einen monoklonalen Antikörper spezifisch für Cytokeratin 5 bzw. 7 und der andere der beiden Rezeptoren $R_2$ oder $R_1$ einen monoklonalen Antikörper spezifisch für CK 19 bzw. 18 enthält, oder beide Rezeptoren einen monoklonalen Antikörper spezifisch für CK 8 enthalten, die feste von der flüssigen Phase trennt und die Markierung in einer der beiden Phasen mißt.

2. Verfahren nach Anspruch 1,
**dadurch gekennzeichnet,**
daß als Rezeptor $R_1$ an eine Festphase gebundene monoklonale Antikörper gegen CK 5, CK 7 oder CK 8 verwendet werden.

3. Verfahren nach Anspruch 2,
**dadurch gekennzeichnet,**
daß eine Festphase, die mit Streptavidin beschichtet ist und als Rezeptor $R_1$ ein Konjugat eines monoklonalen Antikörpers gegen CK 5, CK 7 oder CK 8 und Biotin verwendet wird, wobei die Bindung des monoklonalen Antikörpers an die Festphase durch die Bindung von Biotin an Streptavidin erfolgt.

4. Reagenz zum Nachweis von Leberzirrhose,
**dadurch gekennzeichnet,**
daß es mindestens zwei Rezeptoren $R_1$ und $R_2$ enthält, von denen $R_1$ die Bindung an eine feste Phase vermittelt und $R_2$ markiert ist, wobei entweder $R_1$ oder $R_2$ einen monoklonalen Antikörper spezifisch für CK 5 bzw. CK 7 und der andere der beiden Rezeptoren $R_2$ oder $R_1$ einen monoklonalen Antikörper spezifisch für CK 19 bzw. CK 18 enthält oder beide Rezeptoren $R_1$ und $R_2$ einen monoklonalen Antikörper spezifisch für CK 8 enthalten.

5. Reagenz nach Anspruch 4,
**dadurch gekennzeichnet,**
daß es als Rezeptor $R_1$ einen an eine feste Phase gebundenen monoklonalen Antikörper gegen CK 5 und als Rezeptor $R_2$ einen markierten Antikörper gegen CK 19 enthält.

6. Verwendung einer Hybridom-Zellinie, die monoklonale Antikörper der Subklasse IgGl, spezifisch für Cytokeratin 5 produziert, mit der Hinterlegungsnummer ECACC 89030306, in einem Verfahren nach einem der Ansprüche 1-3.

7. Verwendung einer Hybridom-Zellinie, die monoklonale Antikörper der Subklasse IgGl, spezifisch für Cytokeratin 7 produziert, mit der Hinterlegungsnummer ECACC 89030301, in einem Verfahren nach einem der Ansprüche 1-3.

8. Verwendung einer Hybridom-Zellinie, die monoklonale Antikörper der Subklasse IgGl, spezifisch für Cytokeratin 18 produziert, mit der Hinterlegungsnummer ECACC 89030303, in einem Verfahren nach einem der Ansprüche 1-3.

9. Verwendung einer Hybridom-Zellinie, die monoklonale Antikörper der Subklasse IgGlk, spezifisch für Cytokeratin 19 produziert, mit der Hinterlegungsnummer ECACC 89030304, in einem Verfahren nach einem der Ansprüche 1-3.

10. Verwendung einer Hybridom-Zellinie, die monoklonale Antikörper der Subklasse IgGl, spezifisch für Cytokeratin 8 produziert, mit der Hinterlegungsnummer ECACC 89030302, in einem Verfahren nach einem der Ansprüche 1-3.

11. Verwendung einer Hybridom-Zellinie, die monoklonale Antikörper der Subklasse IgGl, spezifisch für Cytokeratin 5 produziert, mit der Hinterlegungsnummer ECACC 89030305, in einem Verfahren nach einem der Ansprüch 1-3.

**Claims**

1. Process for the detection of liver cirrhosis, characterised in that one incubates a sample of a body fluid with at least two receptors $R_1$ and $R_2$, of which $R_1$ brings about the binding to a solid phase and $R_2$ is labelled, whereby either $R_1$ or $R_2$ contains a monoclonal antibody specific for cytokeratin 5 or 7, respectively, and the other of the two receptors $R_2$ or $R_1$ contains a monoclonal antibody specific for CK 19 or 18, respectively, or both receptors contain a monoclonal antibody specific for CK 8, separates the solid from the liquid phase and measures the labelling in one of the two phases.

2. Process according to claim 1, characterised in that, as receptor $R_1$, monoclonal antibodies against CK 5, CK 7 or CK 8 bound to a solid phase are used.

3. Process according to claim 2, characterised in that a solid phase which is coated with streptavidin is used and, as receptor $R_1$, a conjugate of a monoclonal antibody against CK 5, CK 7 or CK 8 and biotin, whereby the binding of the monoclonal antibody to the solid phase takes place by the binding of biotin to streptavidin.

4. Reagent for the detection of liver cirrhosis, characterised in that it contains at least two receptors $R_1$ and $R_2$, of which $R_1$ brings about the binding to a solid phase and $R_2$ is labelled, whereby either $R_1$ or $R_2$ contains a monoclonal antibody specific for VK 5 or CK 7, respectively, and the other of the two receptors $R_2$ or $R_1$ contains a monoclonal antibody specific for CK 19 or CK 18, respectively, or both receptors $R_1$ and $R_2$ contain a monoclonal antibody specific for CK 8.

5. Reagent according to claim 4, characterised in that, as receptor $R_1$, it contains a monoclonal antibody against CK 5 bound to a solid phase and, as receptor $R_2$, a labelled antibody against CK 19.

6. Use of a hybridoma cell line which produces monoclonal antibodies of the subclass IgGl specific for cytokeratin 5 with the deposit number ECACC 89030306 in a process according to one of claims 1 - 3.

7. Use of a hybridoma cell line which produces monoclonal antibodies of the subclass IgGl specific for cytokeratin 7 with the deposit number ECACC 89030301 in a process according to one of claims 1 - 3.

8. Use of a hybridoma cell line which produces monoclonal antibodies of the subclass IgGl specific for cytokeratin 18 with the deposit number ECACC 89030303 in a process according to one of claims 1 - 3.

9. Use of a hybridoma cell line which produces monoclonal antibodies of the subclass IgGk specific for cytokeratin 19 with the deposit number ECACC 89030304 in a process according to one of claims 1 - 3.

10. Use of a hybridoma cell line which produces monoclonal antibodies of the subclass IgGl specific for cytokeratin 8 with the deposit number ECACC 89030302 in a process according to one of claims 1 - 3.

11. Use of a hybridoma cell line which produces monoclonal antibodies of the subclass IgGl specific for cytokeratin 5 with the deposit number ECACC 89030305 in a process according to one of claims 1 - 3.

**Revendications**

1. Procédé pour la mise en évidence de la cirrhose du foie, caractérisé en ce que l'on incube l'échantillon d'un liquide biologique avec au moins deux récepteurs $R_1$ et $R_2$ parmi lesquels $R_1$ permet la liaison à une phase solide et $R_2$ est marqué, $R_1$ ou $R_2$ contenant un anticorps monoclonal spécifique de la cytokératine 5 ou 7 et l'autre des deux récepteurs $R_2$ ou $R_1$ contenant un anticorps monoclonal spécifique de CK 19 ou 18, ou bien les deux récepteurs contenant un anticorps monoclonal spécifique de CK 8, on sépare la phase solide de la phase liquide et on mesure le marqueur dans l'une des deux phases.

2. Procédé selon la revendication 1, caractérisé en ce que l'on utilise comme récepteur $R_1$ des anticorps monoclonaux contre CK 5, CK 7 ou CK 8 liés à une phase solide.

3. Procédé selon la revendication 2, caractérisé en ce que l'on utilise une phase solide qui est recouverte de streptavidine et comme récepteur $R_1$ un conjugué d'un anticorps monoclonal contre CK 5, CK 7 ou CK 8 et de biotine, la liaison de l'anticorps monoclonal à la phase solide étant réalisée par le biais de la liaison de la biotine à la streptavidine.

4. Réactif pour la mise en évidence de la cirrhose du foie, caractérisé en ce qu'il contient au moins deux récepteurs $R_1$ et $R_2$ parmi lesquels $R_1$ permet la liaison à une phase solide et $R_2$ est marqué, $R_1$ ou $R_2$ contenant un anticorps monoclonal spécifique de CK 5 ou CK 7 et l'autre des deux récepteurs $R_2$ ou $R_1$ contenant un anticorps monoclonal spécifique de CK 19 ou CK 18, ou bien les deux récepteurs $R_1$ et $R_2$ contenant un anticorps monoclonal spécifique de CK 8.

5. Réactif selon la revendication 4, caractérisé en ce qu'il contient comme récepteur $R_1$ un anticorps mo-

noclonal contre CK 5 lié à une phase solide et comme récepteur $R_2$ un anticorps marqué contre CK 19.

6. Utilisation d'une lignée cellulaire d'hybridome qui produit des anticorps monoclonaux de la sous-classe IgGI, spécifiques de la cytokératine 5, de numéro de dépôt ECACC 89030306, dans un procédé selon l'une des revendications 1 à 3.

7. Utilisation d'une lignée cellulaire d'hybridome qui produit des anticorps monoclonaux de la sous-classe IgGI, spécifiques de la cytokératine 7, de numéro de dépôt ECACC 89030301, dans un procédé selon l'une des revendications 1 à 3.

8. Utilisation d'une lignée cellulaire d'hybridome qui produit des anticorps monoclonaux de la sous-classe IgGI, spécifiques de la cytokératine 18, de numéro de dépôt ECACC 89030303, dans un procédé selon l'une des revendications 1 à 3.

9. Utilisation d'une lignée cellulaire d'hybridome qui produit des anticorps monoclonaux de la sous-classe IgGIk, spécifiques de la cytokératine 19, de numéro de dépôt ECACC 89030304, dans un procédé selon l'une des revendications 1 à 3.

10. Utilisation d'une lignée cellulaire d'hybridome qui produit des anticorps monoclonaux de la sous-classe IgGI, spécifiques de la cytokératine 8, de numéro de dépôt ECACC 89030302, dans un procédé selon l'une des revendications 1 à 3.

11. Utilisation d'une lignée cellulaire d'hybridome qui produit des anticorps monoclonaux de la sous-classe IgGI, spécifiques de la cytokératine 5, de numéro de dépôt ECACC 89030305, dans un procédé selon l'une des revendications 1 à 3.